# EUROPEAN PATENT APPLICATION

(11) **EP 4 834 210 A1**
(43) Date of publication of application: **04.11.2026**
(21) Application number: 23963010.6
(22) Date of filing: 25.12.2023
(51) Int. Cl.: A61B 5/0215

(54) **SENSOR-EQUIPPED MEDICAL DEVICE**

(71) Applicant: ASAHI INTECC CO., LTD., Seto-shi, Aichi, 489-0071 (JP)
(72) Inventor: NAMIMA, Satoshi, Seto-shi, Aichi 489-0071 (JP); KOMATSU, Hiroyuki, Seto-shi, Aichi 489-0071 (JP); HAMADA, Takefumi, Seto-shi, Aichi 489-0071 (JP)
(74) Representative: Prinz & Partner mbB
(86) International application number: PCT/JP2023/046449
(87) International publication number: WO 2025/141649

(57) **Abstract**

A medical device with a sensor comprises a sensor, a core shaft, and wiring with a transverse sectional shape in which the thickness direction is smaller than the width direction, wherein a lead electrically connected to the sensor are positioned inside the wiring, the wiring being wound around the core shaft in a predetermined range of the long axis direction of the core shaft in a state where the thickness direction continuously follows the radial direction of the core shaft.

## Description

### TECHNICAL FIELD

The present disclosure relates to a medical device with a sensor.

### BACKGROUND ART

Medical devices with sensors being attached to their distal end portions are known. For example, Patent Literature 1 discloses an intravascular device having a sensing element, a core member, a plurality of communication lines wound around the core member, and a polymer jacket. Hereinafter, the core member will also be referred to as a "core shaft." The communication lines will also be referred to as "wiring."

### Citation List

### Patent Literature

[Patent Literature 1] JP 2018-516623 W

### SUMMARY OF INVENTION

### TECHNICAL PROBLEM

When such a medical device with a sensor is passed through the curved portion of a blood vessel, it is bent so as to follow the curved shape of the blood vessel. When bending stress is applied to the medical device with a sensor, problems that the wiring is twisted and that the wiring is pulled are caused due to the core shaft being bent. Such problems are not considered at all in the intravascular device described in Patent Literature 1.

The present disclosure was made to solve at least a part of the problems mentioned above and aims to reduce loads on wiring in a medical device with a sensor.

### SOLUTION TO PROBLEM

The present disclosure was made to solve at least a part of the problems mentioned above, and can be implemented as the following aspects.
(1) According to one aspect of the present disclosure, a medical device with a sensor is provided. This medical device with a sensor comprises a sensor, a core shaft, and wiring with a transverse sectional shape in which the thickness direction is smaller than the width direction, wherein a lead electrically connected to the sensor are positioned inside the wiring, the wiring being wound around the core shaft in a predetermined range in the long axis direction of the core shaft in a state where the thickness direction continuously follows the radial direction of the core shaft.
   According to this configuration, the wiring is wound around the core shaft in a state where the thickness direction continuously follows the radial direction of the core shaft. Consequently, even if bending stress is applied to the medical device with a sensor and the core shaft is bent, the wiring is less likely to rotate on the outer peripheral surface of the core shaft, thereby inhibiting the wiring from twisting. As a result, loads on the wiring during use of the medical device with a sensor can be reduced.
(2) In the medical device with a sensor of the above aspect, the core shaft may comprise, in at least a part of the predetermined range, tapered portions which taper in outer diameter from the proximal end toward the distal end. According to this configuration, gradually variable rigidity of the core shaft can be realized by the tapered portions. The tapered portions are provided in at least a part of the predetermined range where the wiring is wound around the core shaft. Therefore, even if the core shaft is bent, tensile loads on the wiring can be reduced because the wiring is moved to the distal end side of the tapered portions. To this end, loads on the wiring can be reduced during use of the medical device with a sensor.
(3) In the medical device with a sensor of the above aspect, the wiring may be wound around the tapered portions of the core shaft. According to this configuration, even if the core shaft is bent, tensile loads on the wiring can be reduced because the wiring is moved to the distal end side of the tapered portions. To this end, loads on the wiring can be reduced during use of the medical device with a sensor.
(4) In the medical device with a sensor of the above aspect, it may further comprise coils which cover at least a part of the core shaft in the long axis direction of the core shaft. According to this configuration, the torquability of the medical device with a sensor can be improved because it comprises the coils.
(5) In the medical device with a sensor of the above aspect, the wound direction of the coil may be opposite to the wound direction of the wiring. According to this configuration, when the medical device with a sensor is rotated, the direction in which the coil is compressed can be opposite to the direction in which the wiring is compressed. For this reason, imbalances in performance of the medical device with a sensor to be rotated and operated can be reduced to improve the torquability of the medical device with a sensor.
(6) In the medical device with a sensor of the above aspect, the core shaft is a first core shaft, the medical device may further comprise a second core shaft positioned on the more proximal end side than the first core shaft with a gap being made and positioned between the distal end of the second core shaft and the proximal end of the first core shaft. According to this configuration, the structurally sophisticated medical device with a sensor can be easily manufactured because it has the first core shaft and the second core shaft. The first core shaft and the second core shaft can be manufactured by using different materials.
(7) In the medical device with a sensor of the above aspect, it may further comprise a tube covering a portion of the first core shaft and at least a portion of the second core shaft, and connecting the first core shaft to the second core shaft. According to this configuration, the torquability can be improved by virtue of the first core shaft and the second core shaft being connected via the tube. The tube can block noise external to the medical device with a sensor to improve the accuracy of signals transmitted through the wiring.
(8) In the medical device with a sensor of the above aspect, the lead is a first lead, and the wiring may have a second lead tied together with the first lead. According to this configuration, two signals outputted from the sensor can be transmitted because the wiring has the second lead tied together with the first lead.
(9) In the medical device with a sensor of the above aspect, the wiring may have a pair of opposing main surfaces and a pair of opposing side surfaces, and the wiring may be wound around the core shaft in a state where a first main surface of the pair of main surfaces continuously faces the outer peripheral surface of the core shaft. According to this configuration, the wiring is wound around the core shaft in a state where the first main surface of the pair of main surfaces faces the outer peripheral surface of the core shaft. Therefore, even if the core shaft is bent, the wiring is less likely to rotate on the surface of the core shaft, thereby inhibiting the wiring from twisting.
(10) In the medical device with a sensor of the above aspect, the wiring may be slidable with respect to the core shaft in at least a part of the predetermined range. According to this configuration, the wiring is slidable relative to the core shaft in at least a portion of the predetermined range in which the wiring is wound around the core shaft. For this reason, because the wiring is moved while the core shaft is bent, tensile loads on the wiring can be reduced.
(11) In the medical device with a sensor of the above aspect, the wiring may further have coating portions which cover the outer peripheral surfaces of the lead. According to this configuration, the lead can be electrically insulated by the coating portions.
(12) In the medical device with a sensor of the above aspect, the core shaft may pass through the sensor in the long axis direction. According to this configuration, rotational and compressive forces applied to the medical device with a sensor can be easily transmitted to the sensor in the distal end portion.
(13) In the medical device with a sensor of the above aspect, it may further have an adhesive layer which bonds the core shaft to the wiring. According to this configuration, at least a portion of the wiring can be secured to the core shaft by the adhesive layer.

The present disclosure may be realized in various aspects; it can be realized with aspects such as a medical device with a sensor, parts of a core shaft and wiring for the medical device with a sensor, and a catheter including the medical device with a sensor, as well as a method for manufacturing the same, for example.

### BRIEF DESCRIPTION OF THE DRAWINGS

Fig. 1 is an explanatory view illustrating the configuration of a medical device with a sensor.
Fig. 2 is an enlarged sectional view of the distal end side of the medical device with a sensor.
Fig. 3 is an explanatory view illustrating the configuration of a tubular member.
Fig. 4 is an explanatory view illustrating the configuration of a sensor sheet.
Fig. 5 is an explanatory view explaining a method for making a sensor.
Fig. 6 is an explanatory view illustrating the configuration of wiring.
Fig. 7 is an enlarged view of the proximal end side in the medical device with a sensor.
Fig. 8 is an explanatory view illustrating the configuration of a connector.
Fig. 9 is an explanatory view illustrating the configuration of the proximal end portion in the wiring.
Fig. 10 is an enlarged view of the wiring which is wound around a first core shaft.
Fig. 11 is a view illustrating an example of the transverse section in the line A-A of Fig. 10.
Fig. 12 is a view illustrating another example of the transverse section in the line A-A of Fig. 10.
Fig. 13 is an explanatory view illustrating the configuration of a medical device with a sensor in accordance with a second embodiment.
Fig. 14 is an explanatory view illustrating the configuration of wiring in accordance with a third embodiment.
Fig. 15 is an explanatory view illustrating the configuration of a medical device with a sensor in accordance with a fourth embodiment.
Fig. 16 is an explanatory view illustrating the configuration of a medical device with a sensor in accordance with a fifth embodiment.
Fig. 17 is an explanatory view illustrating the configuration of a medical device with a sensor in accordance with a sixth embodiment.
Fig. 18 is an explanatory view illustrating the configuration of a medical device with a sensor in accordance with a seventh embodiment.
Fig. 19 is a view illustrating an example of the transverse section in the line A-A of Fig. 10 in accordance with an eighth embodiment.

### DESCRIPTION OF EMBODIMENTS

### <First Embodiment>

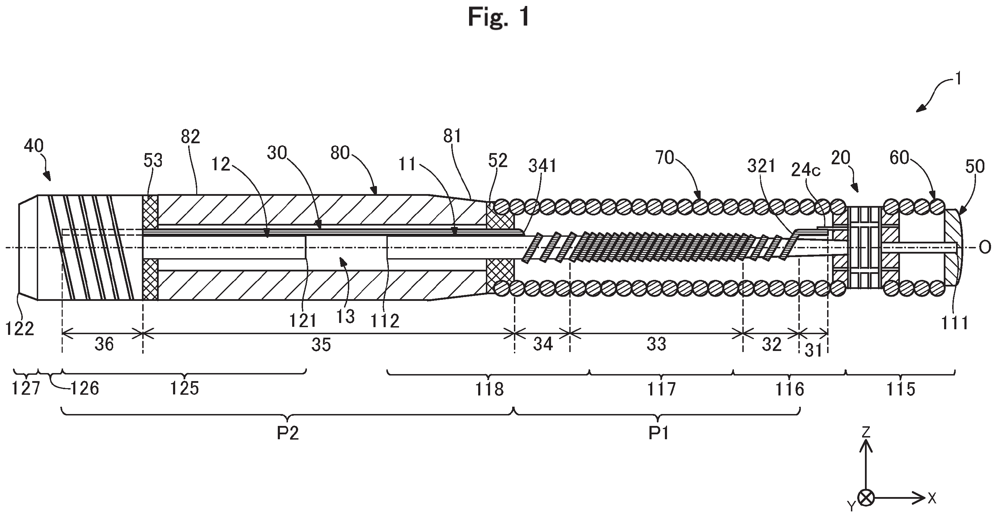

Fig. 1 is an explanatory view illustrating the configuration of a medical device with a sensor 1. Fig. 1 illustrates a sensor 20 and a connector 40 in the external configurations, and other portions in the longitudinal sectional configurations. The medical device with a sensor 1 is a device which is inserted into and used in a cerebral blood vessel to measure the electrical resistance of bodily fluids such as blood flowing through the vessel, for example. The medical device with a sensor 1 is a guide wire with a sensor. The electrical resistance detected by the medical device with a sensor 1 is used, for example, to determine the type of a blood clot generated in the cerebral blood vessel.

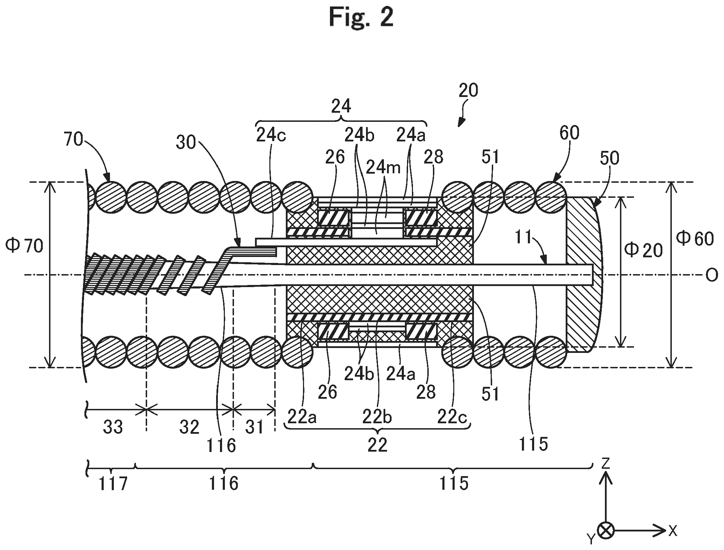

In Fig. 1, the axis passing through the center of the medical device with a sensor 1 is represented by the axis line O. Hereinafter, for each member in the medical device with a sensor 1, the center axis at least passing through the centers of a first core shaft 11, a second core shaft 12, a first coil 60, a second coil 70, a tube 80, and first to sixth tubes 41a-41f will be described as coinciding with the axis line O. The center axis passing through the center of each member in the medical device with a sensor 1 may differ from the axis line O. Fig. 1 illustrates XYZ-axes that are orthogonal to each other. The X-axis corresponds to the longitudinal direction of the medical device with a sensor 1; in other words, it corresponds to the long axis direction of the medical device with a sensor 1. The Y-axis corresponds to the width direction of the medical device with a sensor 1. The Z-axis corresponds to the height direction of the medical device with a sensor 1. The +X-axis direction in Fig. 1 is referred to as the "distal end side" of the medical device with a sensor 1 and each component member. The -X-axis direction in Fig. 1 is referred to as the "proximal end side" of the medical device with a sensor 1 and each component member. With regard to the medical device with a sensor 1 and each component member, one end located on the distal end side is referred to as the "distal end," and the other end located on the proximal end side is referred to as the "proximal end." For the medical device with a sensor 1 and each component member, the distal end and its vicinity are referred to as the "distal end portion," and the proximal end and its vicinity are referred to as the "proximal end portion." The distal end side is inserted into a living body, and the proximal end side is operated by an operator such as a doctor. These points are also common to Fig. 2 and subsequent figures. In the present embodiment, expressions "the same" and "equal" indicate much the same, and allow variances resulting from manufacturing tolerances or the like. In the present embodiment, the expression "approximately constant" indicates "nearly constant," and allows variances resulting from manufacturing tolerances or the like.

The medical device with a sensor 1 comprises the first core shaft 11, the second core shaft 12, a sensor 20, wiring 30, a connector 40, a distal tip 50, the first coil 60, the second coil 70, and the tube 80.

The first core shaft 11 is a solid member which has an elongated shape extending along the X-axis direction. The first core shaft 11 has, in order from a distal end 111 toward a proximal end 112, a first portion 115, a second portion 116, a third portion 117, and a fourth portion 118. The outer peripheral surface of the first core shaft 11 is coated by a first insulating tube 15 which has insulation properties. In other words, the first core shaft 11 includes the first insulating tube 15.

The first portion 115 is a portion provided on the most distal end side of the first core shaft 11. The first portion 115 is a portion where press processes have been applied to the smallest outer diameter portion in the first core shaft 11. The number of press processes and the extent of presses with respect to the first portion 115 may be determined optionally. The second portion 116 is a portion provided between the first portion 115 and the third portion 117. The distal end of the second portion 116 has the same outer diameter as that of the proximal end of the first portion 115. The proximal end of the second portion 116 has the same outer diameter as that of the distal end of the third portion 117. The second portion 116 has a gradually tapered shape which gradually decreases in outer diameter from the proximal end toward the distal end. The third portion 117 is a portion provided between the second portion 116 and the fourth portion 118. The distal end of the third portion 117 has the same outer diameter as that of the proximal end of the second portion 116. The proximal end of the third portion 117 has the same outer diameter as that of the distal end of the fourth portion 118. The third portion 117 has a gradually tapered shape which gradually decreases in outer diameter from the proximal end toward the distal end. The inclination of the taper in the third portion 117 is greater than that of the taper in the second portion 116. The second portion 116 is a tapered portion. The third portion 117 is a tapered portion. The fourth portion 118 is a portion provided on the most proximal end side of the first core shaft 11. The fourth portion 118 is a portion having the largest outer diameter in the first core shaft 11, and has a substantially cylindrical shape with a substantially constant outer diameter. The outer diameters, the lengths in the axis line O direction, and the transverse sectional shapes of the first, second, third, and fourth portions 115, 116, 117, 118 may be determined optionally.

The second core shaft 12 is a solid member which has an elongated shape extending along the X-axis direction. The second core shaft 12 is positioned on the more proximal end side than and coaxially with the first core shaft 11. A gap 13 is formed between the first core shaft 11 and the second core shaft 12. The gap 13 is formed with the proximal end face of the first core shaft 11 and the distal end face of the second core shaft 12 being positioned apart from one another. The second core shaft 12 has a fifth portion 125, a sixth portion 126, and a seventh portion 127 from a distal end 121 toward a proximal end 122 in order. The outer peripheral surface of the second core shaft 12 is coated by a second insulating tube 16 which has insulation properties. In other words, the second core shaft 12 includes the second insulating tube 16.

The fifth portion 125 is a portion provided on the most distal end side of the second core shaft 12. The fifth portion 125 is a portion having the least outer diameter in the second core shaft 12, and has a substantially cylindrical shape with a substantially constant outer diameter. The sixth portion 126 is a portion provided between the fifth portion 125 and the sixth portion 126. The distal end of the sixth portion 126 has the same outer diameter as that of the proximal end of the fifth portion 125. The proximal end of the sixth portion 126 has the same outer diameter as that of the distal end of the seventh portion 127. The sixth portion 126 has a gradually tapered shape which gradually decreases in outer diameter from the proximal end toward the distal end. The seventh portion 127 is a portion provided on the most proximal end side of the second core shaft 12. The seventh portion 127 has a gradually tapered shape which gradually expands in outer diameter from the proximal end toward the distal end. The seventh portion 127 is externally exposed from the proximal end of the connector 40. The outer diameters, the lengths in the axis line O direction, and the transverse sectional shapes of the fifth, sixth and seventh portions 125, 126 and 127 may be determined optionally.

The first coil 60 and the second coil 70 are cylindrical coils which have flexibility and have substantially constant outer diameters from the proximal ends toward the distal ends. The first coil 60 and the second coil 70 are multi-thread coils formed by a plurality of wires wound in a multi-thread manner. The first coil 60 is positioned on the more distal end side than the sensor 20. The first coil 60 surrounds the outer periphery of the first portion 115 of the first core shaft 11 which protrudes from the sensor 20 toward the distal end side. The second coil 70 is positioned on the more proximal end side than the sensor 20. The second coil 70 surrounds the outer peripheries of the second portion 116, the third portion 117 and a portion of the distal end side of the fourth portion 118 in the first core shaft 11. The first coil 60 and the second coil 70 can be formed from any material. The first coil 60 and the second coil 70 can be formed by using at least any one of stainless steel alloys, superelastic alloys, radiolucent alloys, and radiopaque alloys. As stainless steel alloys, SUS 304 and SUS 316 can be exemplified. As superelastic alloys, nickel-titanium and nickel-titanium alloys can be exemplified. As radiolucent alloys, piano wire, nickel-chromium based alloys, and cobalt alloys can be exemplified. As radiopaque alloys, gold, platinum, tungsten, and alloys containing these elements can be exemplified.

The first coil 60 and the second coil 70 may also be single-thread coils formed by a single wire being wound in a single-thread manner. The first coil 60 and the second coil 70 may also be single-thread strand coils formed by winding in a single-thread manner a strand made of a plurality of wires being stranded. The first coil 60 and the second coil 70 may also be multi-thread strand coils formed by using a plurality of strands made of a plurality of wires being stranded to wind each strand in a multi-thread manner. The types of the first coil 60 and the second coil 70 may be the same or different. The outer and inner diameters of the first coil body 60 and the second coil body 70 can be determined arbitrarily.

The tube 80 has a substantially cylindrical shape. The tube 80 has a distal end portion 81 and a main body part 82. The distal end portion 81 is a tapered-shaped portion which gradually decreases in outer diameter from the proximal end toward the distal end. The main body part 82 is a portion having a substantially constant outer diameter. The tube 80 has an inner cavity with a substantially constant inner diameter. Through the inner cavity of the tube 80, a portion of the proximal end side of the first core shaft 11, a portion of the distal end side of the second core shaft 12, and the wiring 30 are passed. The gap 13 is provided in the inner cavity of the tube 80. The tube 80 improves the rigidity of the medical device with a sensor 1 as the tube 80 is secured to the first core shaft 11 and the second core shaft 12. In other words, the tube 80 covers a portion of the proximal end side of the first core shaft 11 and a portion of the distal end side of the second core shaft 12 to connect the first core shaft 11 to the second core shaft 12. The tube 80 may be formed using at least any one of nickel-titanium and nickel-titanium alloys, for example.

The distal tip 50 is a member that connects the distal end 111 of the first core shaft 11 to the distal end of the first coil 60. A second joint part 52 is a member that joins the proximal end of the first coil 60, the distal end of the tube 80, and a portion of the first core shaft 11. A third joint part 53 is a member that joins the proximal end of the tube 80, the connector 40, and a portion of the second core shaft 12. The distal tip 50, the second joint part 52, and the third joint part 53 can be formed using any suitable bonding agent. As the bonding agent, at least any one of metal solders such as silver solder, gold solder, zinc, SnAg alloys, and Au-Sn alloys, and adhesives such as epoxy-based adhesives can be used.

Fig. 2 is an enlarged sectional view of the distal end side of the medical device with a sensor 1. The sensor 20 shown in Fig. 2 is positioned at the distal end portion of the medical device with a sensor 1. The sensor 20 is a structure that includes a sensor element for measuring the electrical resistance of bodily fluids such as blood flowing through a blood vessel. Within the sensor 20, the first portion 115 of the first core shaft 11 is passed through. In other words, the first core shaft 11 passes through the sensor 20 in the X-axis direction. The sensor 20 comprises a tubular member 22, a sensor sheet 24, spacers 26, 28, and a first joint part 51.

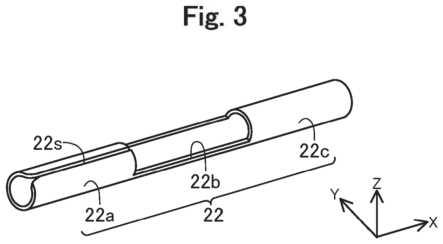

Fig. 3 is an explanatory view illustrating the configuration of the tubular member 22. The tubular member 22 has a proximal end cylindrical portion 22a, a middle connection portion 22b, and a distal end cylindrical portion 22c. The proximal end cylindrical portion 22a is a substantially cylindrical portion located on the proximal end side in the tubular member 22. On the side surface of the proximal end cylindrical portion 22a, a linear slit 22s which passes through the inner and outer sides of the cylinder is formed. The intermediate connecting portion 22b is a portion provided between the proximal end cylindrical portion 22a and the distal end cylindrical portion 22c. The transverse section of the intermediate connecting portion 22b is semicircular. In other words, at the intermediate connection portion 22b, the inner surface of the cylinder is externally exposed. The distal end cylindrical portion 22c is a substantially cylindrical portion located on the distal end side in the tubular member 22. No slit is formed on the side surface of the distal end cylindrical portion 22c. The tubular member 22 can be formed from any resin material.

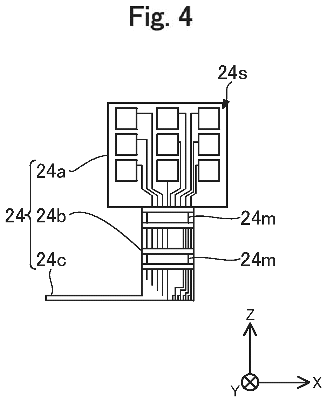

Fig. 4 is an explanatory view illustrating the configuration of the sensor sheet 24. The sensor sheet 24 is a sheet-form member. The sensor sheet 24 has a sensor array 24s, microchips 24m, a large width portion 24a, a small width portion 24b, and sensor wiring 24c.

The sensor array 24s includes nine sensor elements positioned in a grid pattern. The sensor elements are elements that measure the electrical resistance of bodily fluids such as blood flowing through a blood vessel. The microchips 24m transmit signals representing the values measured by the sensor array 24s. The large width portion 24a is a wide, strip-shaped sheet, and the sensor array 24s is positioned on its surface. The small width portion 24b is a strip-shaped sheet that is narrower than the large width portion 24a, and the microchips 24m are positioned on its surface. The sensor wiring 24c is wiring that transmits signals sent by the microchips 24m. The sensor wire 24c is electrically connected to the distal end portion of the wiring 30.

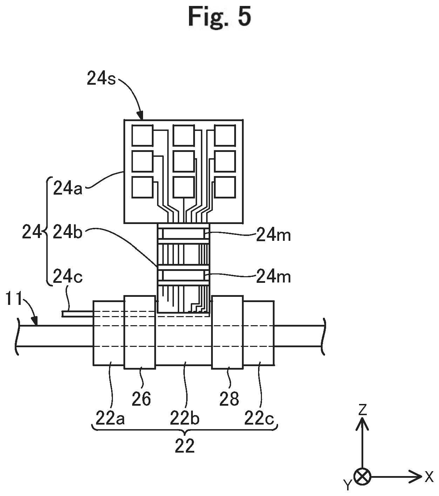

Fig. 5 is an explanatory view explaining a method for making the sensor 20. A worker can make the sensor 20 with procedures as follows. The worker prepares the tubular member 22, the sensor sheet 24, the spacer 26, and the spacer 28. The spacers 26, 28 are annular members having slightly larger inner diameters than the tubular member 22. The spacers 26, 28 may be formed from any resin material. The worker inserts the sensor sheet 24 into the inner side of the tubular member 22 via the slit 22s. Then, as shown in Fig. 5, the sensor sheet 24 is brought about such a state that the large width portion 24a and the small width portion 24b externally protrude from the tubular member 22. The worker inserts the distal end portion of the first core shaft 11 into the inner side of the tubular member 22. The worker fits the annular spacer 26 into the tubular member 22 from the end of the proximal end cylindrical portion 22a side. The worker fits the annular spacer 28 into the tubular member 22 from the end of the distal end cylindrical portion 22c side. Then, as shown in Fig. 5, the tubular member 22 and the sensor sheet 24 are held in the state shown in Fig. 5 by the annular spacers 26, 28. The worker wraps the sensor sheet 24 around the tubular member 22 and the spacers 26, 28. As a result, the small width portion 24b in the sensor sheet 24 is brought into the arrangement that covers the outer periphery of the intermediate connection portion 22b. As shown in Fig. 2, when the sensor sheet 24 is wrapped, one microchip 24m and another microchip 24m on the small width portion 24b are positioned around the tubular member 22 with those being stacked one on top of the other. The large width portion 24a in the small width portion 24b is wrapped around the outer periphery of the spacers 26, 28. As a result, nine sensor elements of the sensor array 24s are positioned at the outermost periphery of the sensor 20. In other words, three sensor elements are positioned at 120° intervals along the circumferential direction.

Returning to Fig. 2, description is continued. The first joint part 51 is a member that joins the sensor 20, the first core shaft 11, the first coil 60, and the second coil 70. The first joint part 51 is positioned within the tubular member 22 of the sensor 20 and joins the sensor 20 to the first core shaft 11. The first joint part 51 is positioned at the outer peripheral portion on the more distal end side than the spacer 28 of the sensor 20, and joins the sensor 20 to the first coil 60. The first joint part 51 is positioned at the outer peripheral portion on the more proximal end side than the spacer 26 of the sensor 20, and joins the sensor 20 to the second coil 70. The first joint part 51 can be formed by any bonding agent. As the bonding agent, at least any one of metal solders such as silver solder, gold solder, zinc, SnAg alloys, and Au-Sn alloys, and adhesives such as epoxy-based adhesives can be used. As shown in Fig. 2, the outer diameter Φ60 of the first coil 60 is equal to the outer diameter Φ70 of the second coil 70. The outer diameter Φ20 of the sensor 20 is smaller than the outer diameter Φ60 of the first coil 60. The outer diameter Φ20 of the sensor 20 is smaller than the outer diameter Φ70 of the second coil 70.

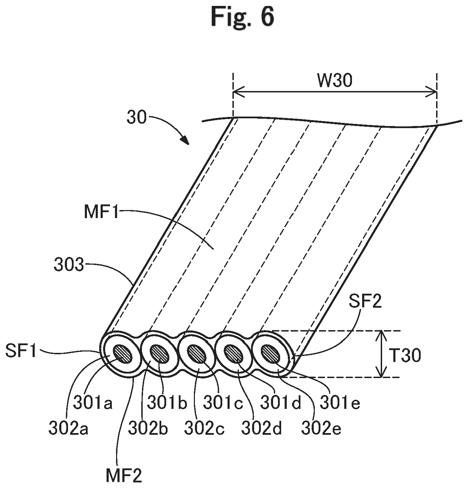

Fig. 6 is an explanatory view illustrating the configuration of the wiring 30. The wiring 30 is a connection line that electrically connects the sensor 20 to the connector 40. As shown in Fig. 1, the distal end portion 31 of the wiring 30 is electrically connected to the sensor 20. The proximal end portion 36 of the wiring 30 is electrically connected to the connector 40. The wiring 30 transmits signals from the sensor 20 to an external device via the connector 40. As shown in Fig. 6, the wiring 30 comprises leads 301a-301e, inner coating portions 302a-302e, and an outer coating portion 303. The leads 301a-301e are a first lead 301a, a second lead 301b, a third lead 301c, a fourth lead 301d, and a fifth lead 301e. The wiring 30 comprises the first lead 301a. The wiring 30 comprises the second lead 301b. The wiring 30 comprises the third lead 301c. The wiring 30 comprises the fourth lead 301d. The wiring 30 comprises the fifth lead 301e. The inner coating portions 302a-302e are coating portions. The outer coating portion 303 is a coating portion.

The leads 301a-301e are conductive metal conductors. The inner coating portions 302a-302e are tubes formed from insulating resins. The inner coating portion 302a covers the outer peripheral surface of the lead 301a. Similarly, the inner coating portions 302b-302e cover the outer peripheral surfaces of the leads 301b-301e, respectively. The inner coating portions 302a-302e can be formed from insulating resins, for example, polyimide resins. The five leads 301a-301e are positioned horizontally in a single row with each being covered by the inner coating portions 302a-302e. The outer coating portion 303 covers and integrally holds outer peripheral surfaces of the leads 301a-301e and the inner coating portions 302a-302e. The outer coating portion 303 can be formed from insulating resins, for example, urethane resins.

In the wiring 30, the direction in which the leads 301a-301e and the inner coating portions 302a-302e are lined is also referred to as the "width direction." In the wiring 30, the direction perpendicular to both the length and width directions is also referred to as the "thickness direction." As shown in Fig. 6, the wiring 30 has a length T30 in the thickness direction which is smaller than a length W30 in the width direction. The wiring 30 has a flat shape with a transverse sectional shape in which the thickness direction is smaller than the width direction. The transverse section of the wiring 30 is non-circular. Of the side surfaces of the wiring 30, as for a pair of main surfaces having relatively large areas, one is also referred to as a "first main surface MF1" and the other as a "second main surface MF2." The first main surface MF1 and the second main surface MF2 are opposed to each other. In the wiring 30, as for a pair of side surfaces having relatively small areas, one is also referred to as a "first side surface SF1," and the other is also referred to as a "second side surface SF2." The first side surface SF1 and the second side surface SF2 are opposed to each other. The first main surface MF1 is sandwiched between the first side surface SF1 and the second side surface SF2. The second main surface MF2 is sandwiched between the first side surface SF1 and the second side surface SF2.

Returning to Fig. 1, description is continued. The distal end portion 31 of the wiring 30 is electrically connected to the sensor wiring 24c of the sensor 20 with the inner coating portions 302a-302e and the outer coating portion 303 being removed. The distal end portion 31 of the wiring 30 is straight. "Electrically connected" means that the connection is provided in a state where current can flow; in other words, it means that the connection is provided in a state where electricity can be conducted.

The wiring 30 is wound spirally around the outer peripheral surface of the first core shaft 11 in a first section P1. The first section P1 is a section located on the more proximal end side than than the sensor lead 24c of the sensor 20, and on the more distal end side than the second joint part 52. The wiring 30 in the first section P1 has a first roll portion 32, a second roll portion 33, and a third roll portion 34. In the first roll portion 32, the wiring 30 is loosely wound around the second portion 116 of the first core shaft 11. In the first roll portion 32, the adjacent spacings of the wiring 30 gradually decrease from the distal end side toward the proximal end side. In the second roll portion 33, the wiring 30 is closely wound around the third portion 117 and the fourth portion 118 of the first core shaft 11. In the third roll portion 34, the wiring 30 is loosely wound around the fourth portion 118 of the first core shaft 11. In the third roll portion 34, the adjacent spacings of the wiring 30 gradually increase from the distal end side toward the proximal end side. In other words, in the first roll portion 32 and the third roll portion 34, the adjacent spacings of the wiring 30 gradually decrease as they approach the second roll portion 33. By virtue of the wiring 30 being wound around the first core shaft 11, the center axis of the first core shaft 11 can be maintained in alignment with the center axis O of the medical device with a sensor 1. As a result, when the medical device with a sensor 1 is in use, stress concentration in a portion of the first core shaft 11 can be suppressed.

In the second section P2, the wiring 30 extends straight along the outer peripheral surfaces of the first core shaft 11 and the second core shaft 12. In other words, in the second section P2, the wiring 30 is not helical. The second section P2 is a section which is located on the more proximal end side than the second joint part 52 and extends to the inner side of the connector 40. The wiring 30 in the second section P2 has a straight portion 35 and a proximal end portion 36. In the straight portion 35, the wiring 30 extends straight along the fourth portion 118 of the first core shaft 11 and the fifth portion 125 of the second core shaft 12. Also in the gap 13, the wiring 30 extends along the long axis direction of the medical device with a sensor 1. At the proximal end portion 36, the wiring 30 is inserted into the inner side of the connector 40 and is electrically connected to the connector 40.

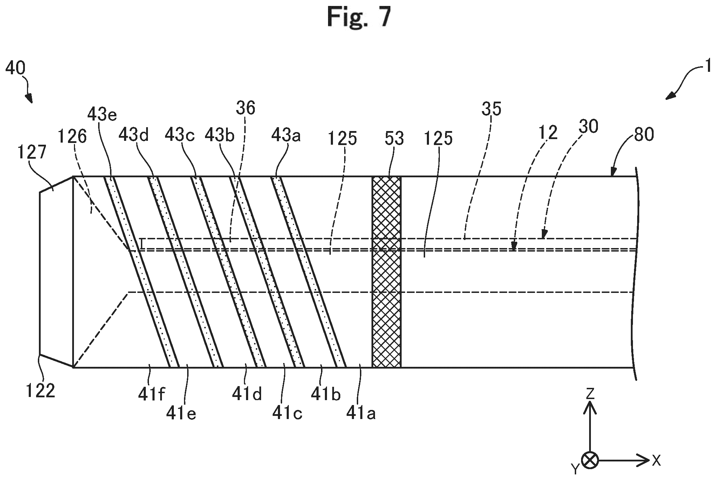

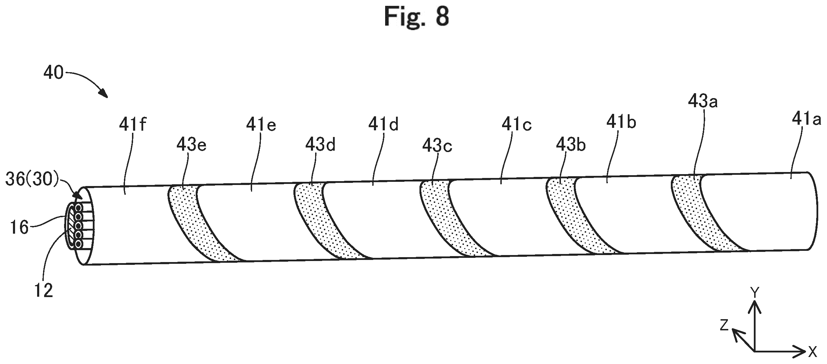

Fig. 7 is an enlarged view of the proximal end side in the medical device with a sensor 1. In Fig. 7, while the appearance of the medical device with a sensor 1 is represented in solid lines, the second core shaft 12 and the wiring 30 which are positioned within the inner cavities of the connector 40 and tube 80 are represented in dashed lines. Fig. 8 is an explanatory view illustrating the configuration of the connector 40. The connector 40 is positioned at the proximal end portion of the medical device with a sensor 1. The connector 40 is a terminal that is connected to an external device that processes output signals from the sensor 20. The second core shaft 12 and the wiring 30 are passed through the inner side of the connector 40. The connector 40 is joined to the proximal end of the tube 80 by the third joint part 53. As shown in Fig. 8, the connector 40 comprises six tubes 41a-41f and five insulators 43a-43e.

The tubes 41a-41f are conductive hollow electrodes. In the present embodiment, tubes 41a-41f are cylindrical. The tubes 41a-41f can be formed from conductive metals, for example, platinum-iridium alloys. The first tube 41a is located on the most distal end side in the connector 40 and on the more proximal end side than the sensor 20. The distal end face of the first tube 41a is vertical to the X-axis direction. The proximal end face of the first tube 41a is inclined with respect to the X-axis direction. The second tube 41b is located on the more proximal end side than the first tube 41a. The distal end face of the second tube 41b is inclined with respect to the X-axis direction. The proximal end face of the second tube 41b is inclined with respect to the X-axis direction. The orientation of tilt and the angle of tilt in the distal end face and the proximal end face of the second tube 41b are the same. The third tube 41c is located on the more proximal end side than the second tube 41b. The fourth tube 41d is located on the more proximal end side than the third tube 41c. The fifth tube 41e is located on the more proximal end side than the fourth tube 41d. The third tube 41c, the fourth tube 41d, and the fifth tube 41e have the same configurations as that of the second tube 41b. The sixth tube 41f is located on the most proximal end side in the connector 40 and on the more proximal end side than the fifth tube 41e. The distal end face of the sixth tube 41f is inclined with respect to the X-axis direction. The proximal end face of the sixth tube 41f is vertical with respect to the X-axis direction.

The insulators 43a-43e are insulating members that insulate adjacent tubes 41a-41f from one another. The insulators 43a-43e can be formed from insulating adhesives, for example, polyimide resins. The first insulator 43a is positioned and sandwiched between the first tube 41a and the second tube 41b. As shown, a portion of the first insulator 43a is externally exposed. "Externally exposed" means that it is visible in appearance. The second insulator 43b is positioned and sandwiched between the second tube 41b and the third tube 41c. A portion of the second conductor 42b is externally exposed. The third insulator 43c is positioned and sandwiched between the third tube 41c and the fourth tube 41d. A portion of the third insulator 43c is externally exposed. The fourth insulator 43d is positioned and sandwiched between the fourth tube 41d and the fifth tube 41e. A portion of the fourth insulator 43d is externally exposed. The fifth insulator 43e is positioned and sandwiched between the fifth tube 41e and the sixth tube 41f. A portion of the fifth insulator 43e is externally exposed.

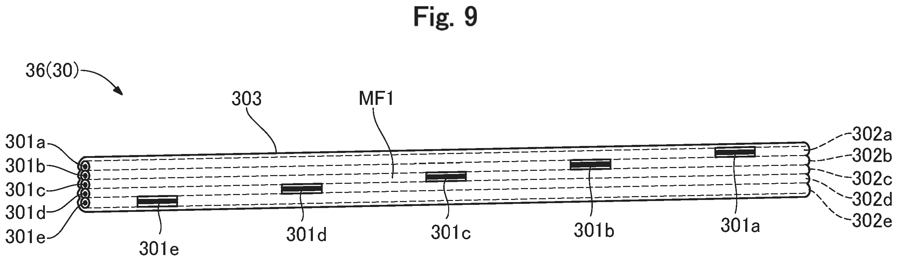

Fig. 9 is an explanatory view illustrating the configuration of the proximal end portion 36 of the wiring 30. As shown in Fig. 7, the proximal end portion 36 of the wiring 30 is positioned within the first to fifth tubes 41a-41e in the connector 40. As for the wiring 30, the inner coating portion 302a and the outer coating portion 303 have been partly peeled off to expose the lead 301a in a position corresponding to the first tube 41a, as shown in Fig. 9. The exposed lead 301a is electrically connected to the first tube 41a. For the wiring 30, the inner coating portion 302b and the outer coating portion 303 have been partly peeled off to expose the lead 301b in a position corresponding to the second tube 41b. The exposed lead 301b is electrically connected to the second tube 41b. For the wiring 30, the inner coating portion 302c and the outer coating portion 303 have been partly peeled off to expose the lead 301c in a position corresponding to the third tube 41c. The exposed lead 301c is electrically connected to the third tube 41c. For the wiring 30, the inner coating portion 302d and the outer coating portion 303 have been partly peeled off to expose the lead 301d in a position corresponding to the fourth tube 41d. The exposed wiring 301d is electrically connected to the fourth tube 41d. For the wiring 30, the inner coating portion 302e and the outer coating portion 303 have been partly peeled off to expose the lead 301e in a position corresponding to the fifth tube 41e. The exposed wiring 301e is electrically connected to the fifth tube 41e. In Fig. 7, the fifth portion 125 of the second core shaft 12 is positioned within the first to fifth tubes 41a-41e in the connector 40. In the connector 40, the sixth portion 126 of the second core shaft 12 is positioned within the sixth tube 41f. The seventh portion 127 of the second core shaft 12 protrudes externally from the proximal end of the connector 40.

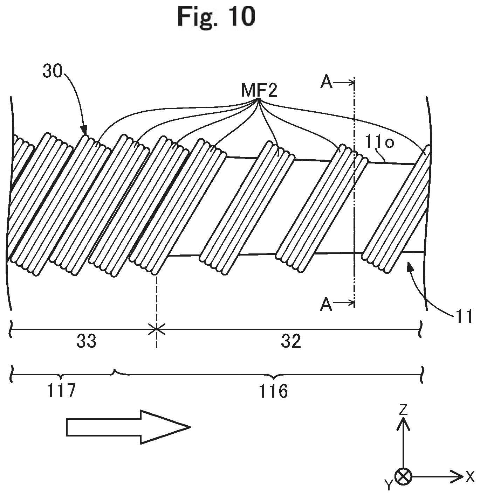

Fig. 10 is an enlarged view of the wiring 30 which is wound around the first core shaft 11. As shown in Fig. 1, the wiring 30 is wound spirally around the outer peripheral surface of the first core shaft 11 in the first section P1. The first section P1 can be a predetermined range in the long axis direction of the medical device with a sensor 1. As shown in Fig. 10, the wiring 30 is wound around the first core shaft 11 with the first main surface MF1 of the pair of main surfaces MF1 and MF2 continuously facing an outer peripheral surface 11o of the first core shaft 11. "Continuously" means that each state of the wiring 30 lined in the X-axis direction on the outer peripheral surface of the first core shaft 11 is the same in its entirety. Namely, the wiring 30 in the first section P1 is in a state where the second main surface MF2 located on the opposite side of the first main surface MF1 is facing outward continuously in appearance.

In the example shown in Fig. 1, the first roll portion 32 of the wiring 30 is located at a position corresponding to a portion of the proximal end side of the second portion 116 in the first core shaft 11. The second roll portion 33 of the wiring 30 is located at a position corresponding to that from the proximal end portion of the second portion 116 to the distal end portion of the fourth portion 118 in the first core shaft 11. In other words, the third portion 117 of the first core shaft 11 is covered by the second roll portion 33 of the wiring 30. The third roll portion 34 of the wiring 30 is located at a position corresponding to a portion of the distal end side of the fourth portion 118 in the first core shaft 11. The second portion 116 and the third portion 117 of the first core shaft 11 are "tapered portions." The wiring 30 is wound around the tapered portions of the first core shaft 11.

The wiring 30 is secured to the first core shaft 11 at two places which are the distal end portion 321 of the first roll portion 32 and the proximal end portion 341 of the third roll portion 34. The wiring 30 and the first core shaft 11 can be secured using any suitable bonding agent such as epoxy-based adhesives. As for the wiring 30, the entire portion of the first roll portion 32 excluding the distal end portion 321, the entire portion of the second roll portion 33, and the entire portion of the third roll portion 34 excluding the proximal end portion 341 are not secured to the first core shaft 11. For this reason, the wiring 30 is slidable relative to the first core shaft 11 in a range from the distal end portion 321 of the first roll portion 32 to the proximal end portion 341 of the third roll portion 34. In other words, the wiring 30 can be moved relative to the first core shaft 11 in the range from the distal end portion 321 of the first roll portion 32 to the proximal end portion 341 of the third roll portion 34, and the relative position of the wiring 30 to the first core shaft 11 can be changed.

In the example shown in Fig. 1, the wiring 30 is configured with a S twist in the first section P1. In this case, the second coil 70 is preferably configured to be wound in the opposite direction to the direction of winding the wiring 30, i.e., with a Z twist. Fig. 1 is merely an example; the wiring 30 may be configured with a Z twist, and the second coil 70 may be configured with a S twist.

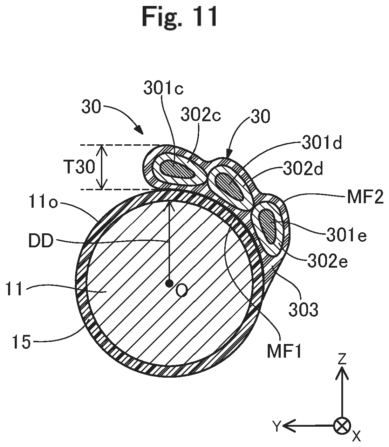

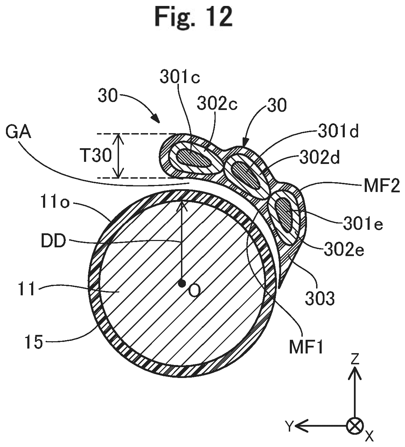

Fig. 11 is a view illustrating an example of the transverse section in the line A-A of Fig. 10. Fig. 12 is a view illustrating another example of the transverse section in the line A-A of Fig. 10. In the first section P1, the wiring 30 is wound around the first core shaft 11 in a state where the thickness direction continuously follows the radial direction DD of the first core shaft 11. The thickness direction of the wiring 30 is a direction indicated by T30 in Figure 11. "The thickness direction follows the radial direction DD" means that the thickness direction generally coincides with the imaginary extension line of the radial direction DD, and that deviations caused by manufacturing tolerances are permitted. As shown in Fig. 11, the first main surface MF1 of the wiring 30 may be in contact with the outer peripheral surface 11o of the first core shaft 11. As shown in Fig. 12, the first main surface MF1 of the wiring 30 may be spaced apart from the outer peripheral surface 11o of the first core shaft 11. In other words, a gap GA may be provided between the first main surface MF1 of the wiring 30 and the outer peripheral surface 11o of the first core shaft 11. The gap GA do not have to be provided continuously. In the first section P1, the wiring 30 may contain mixed parts which are a portion in which the first main surface MF1 is in contact with the outer peripheral surface 11o and a portion in which the gap GA is present. As described above, the first core shaft 11 includes the first insulating tube 15 that has insulating properties. For this reason, the outer peripheral surface of the first insulating tube 15 is represented as the outer peripheral surface 11o of the first core shaft 11 in Figs. 11 and 12.

According to the medical device with a sensor 1 of the first embodiment, the wiring 30 is wound around the first core shaft 11 in a state where the thickness direction continuously follows the radial direction DD of the first core shaft 11. In other words, the wiring 30 is wound around the first core shaft 11 in a state where the first main surface MF1 of the pair of main surfaces MF1 and MF2 continuously faces the outer peripheral surface 11o of the first core shaft 11. Consequently, even if bending stress is applied to the medical device with a sensor 1 and the first core shaft 11 is bent, the wiring 30 is less likely to rotate on the outer peripheral surface 11o of the first core shaft 11, thereby inhibiting the wiring 30 from twisting. As a result, loads on the wiring 30 during use of the medical device with a sensor 1 can be reduced.

According to the medical device with a sensor 1 of the first embodiment, the first core shaft 11 has tapered portions 116, 117. Therefore, gradually variable rigidity of the first core shaft 11 can be realized by the tapered portions 116, 117 of the first core shaft 11. The tapered portions 116, 117 are provided in at least a portion of the predetermined range P1 where the wiring 30 is wound around the core shaft. For this reason, even if the first core shaft 11 is bent, the wiring 30 is moved to the distal end side of the tapered portions 116, 117 and thus tensile loads on the wiring 30 can be reduced. In other words, when the first core shaft 11 is bent, the wiring 30 is moved in a direction indicated by the hollow arrow in Fig. 10. As a result, while the adjacent spacings of the wiring 30 are narrowed in the first roll portion 32, the adjacent spacings of the wiring 30 are widened in the second roll portion 33. In the tapered portions 116, 117, the outer diameter of the first core shaft 11 decreases toward the distal end side. To this end, when the wiring 30 is moved to the distal end side, extra length is created relative to the outer diameter of the first core shaft 11 and thus tensile loads on the wiring 30 can be reduced. As a result, loads on the wiring 30 during use of the medical device with a sensor 1 can be reduced even further.

According to the medical device with a sensor 1 of the first embodiment, the wiring 30 is slidable relative to the first core shaft 11 in at least a portion of the predetermined range P1 in which the wiring 30 is wound around the first core shaft 11. Consequently, because the wiring 30 is moved while the first core shaft 11 is bent, tensile loads on the wiring 30 can be reduced.

According to the medical device with a sensor 1 of the first embodiment, as the wiring 30 has a plurality of tied leads 301a-301e, a plurality of signals outputted from the sensor 20 can be transmitted. According to the medical device with a sensor 1 of the first embodiment, the wiring 30 has inner coating portions 302a-302e and the outer coating portion 303 covering the outer peripheral surfaces of the leads 301a-301e. Therefore, the leads 301a-301e can be electrically insulated by the coating portions 302a-302e, 303.

According to the medical device with a sensor 1 of the first embodiment, the torquability of the medical device with a sensor 1 can be improved because it comprises the first and second coils 60, 70 that cover at least a portion of the first core shaft 11. According to the medical device with a sensor 1 of the first embodiment, the wound direction of the second coil 70 is opposite to that of the wiring 30. For this reason, when the medical device with a sensor 1 is rotated, the direction in which the coil 70 is compressed can be opposite to the direction in which the wiring 30 is compressed. As a result, imbalances in performance when the medical device with a sensor 1 is rotated and operated can be reduced to improve the torquability of the medical device with a sensor 1.

According to the medical device with a sensor 1 of the first embodiment, the first core shaft 11 passes through the sensor 20 in the X-axis direction. Therefore, rotational and compressive forces applied to the medical device with a sensor 1 can be easily transmitted to the sensor 20 in the distal end portion.

According to the medical device with a sensor 1 of the first embodiment, the medical device with a sensor 1 has the first core shaft 11 and the second core shaft 12. Therefore, the portions associated with the first core shaft 11 can be separately made from the portions associated with the second core shaft 12 to easily manufacture the structurally sophisticated medical device with a sensor 1. The first core shaft 11 and the second core shaft 12 can be manufactured by using different materials. According to the medical device with a sensor 1 of the first embodiment, the torquability can be improved by connecting the first core shaft 11 to the second core shaft 12 via the tube 80. The tube 80 can block noise external to the medical device with a sensor 1 to improve the accuracy of signals transmitted through the wiring 30.

### <Second Embodiment>

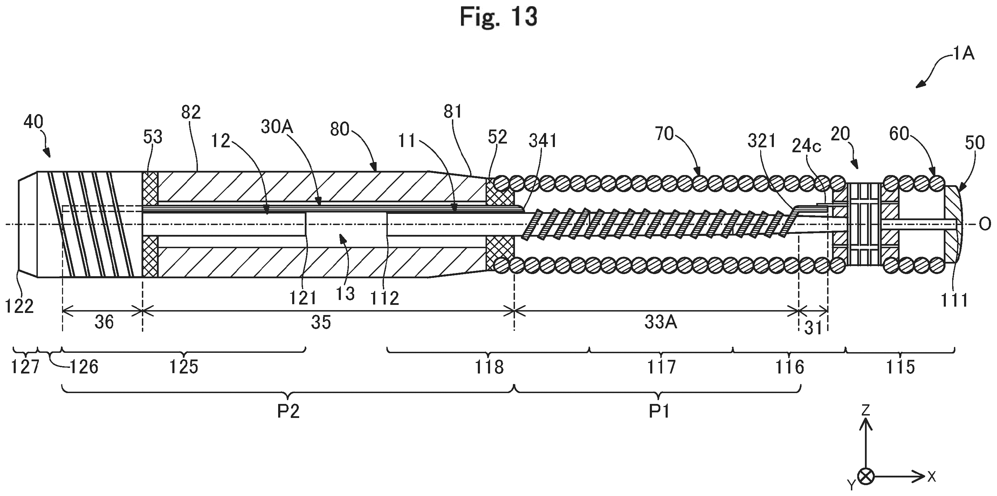

Fig. 13 is an explanatory view illustrating the configuration of a medical device with a sensor 1A in a second embodiment. The medical device with a sensor 1A of the second embodiment comprises wiring 30A instead of the wiring 30 in the configuration described in the first embodiment. The wiring 30A does not have the first roll portion 32 and the third roll portion 34 described in the first embodiment. In other words, the wiring 30A has, from the distal end side toward the proximal end side, the distal end portion 31, a roll portion 33A, a straight portion 35, and the proximal end portion 36. In other words, the wiring 30A in the first section P1 is the roll portion 33A in its entirety.

In the roll portion 33A, the wiring 30A is loosely wound around the first core shaft 11. In other words, in the roll portion 33A, the wiring 30A is wound around the first core shaft 11 with a gap being made between adjacent spacings of the wiring 30A. The adjacent spacings of the wiring 30A are generally even. However, the adjacent spacings of the wiring 30A may also be uneven. Other aspects of the wiring 30A are the same as in the first embodiment. For example, the second embodiment is the same as the first embodiment in that the wiring 30A is wound around the first core shaft 11 in a state where the thickness direction continuously follows the radial direction of the first core shaft 11. It is the same as the first embodiment in that the wiring 30A is slidable between the distal end portion 321 and the proximal end portion 341 relative to the first core shaft 11. The wound directions of the wiring 30A and the second coil 70 are the same as those of the first embodiment.

As such, the configuration of the wiring 30A can be modified in various ways. The wiring 30A in the first section P1 may be loosely wound in its entirety. The wiring 30A in the first section P1 may also be closely wound in its entirety. The wiring 30A may have such a configuration that only any one of the first roll portion 32 and the third roll portion 34 as described in the first embodiment is omitted. The medical device with a sensor 1A of the second embodiment as described above can also achieve the same effects as those of the first embodiment.

### <Third Embodiment>

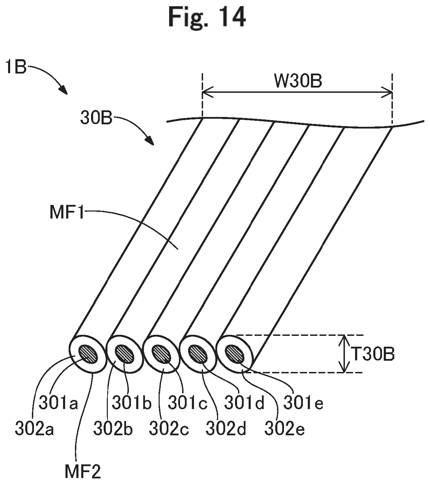

Fig. 14 is an explanatory view illustrating the configuration of wiring 30B in accordance with a third embodiment. A medical device with a sensor 1B of the third embodiment comprises the wiring 30B instead of the wiring 30 in the configuration described in the first embodiment. The wiring 30B does not have the outer coating portion 303 described in the first embodiment.

The wiring 30B is integrally formed of the leads 301a-301e, which are coated by the inner coating portions 302a-302e, by bonding them with a bonding agent. As the bonding agent, any suitable bonding agent such as epoxy-based adhesives can be used. In the wiring 30B, the leads 301a-301e, which are coated by the inner coating portions 302a-302e, may be tied by a tying member to integrate them. In the present embodiment, the inner coating portions 302a-302e are coating portions. Other aspects of the wiring 30B are the same as those in the first embodiment.

As such, the configuration of the wiring 30B can be modified in various ways. In the wiring 30B, the coating portion that coats the outer peripheral surfaces of the leads 301a-301e may be a single layer. In the wiring 30B, the coating portion that coats the outer peripheral surfaces of the leads 301a-301e may have three layers or more. If the coating portion positioned in the outermost periphery of the wiring 30B is made of a material with a low coefficient of friction, the wiring 30B can be moved more smoothly when the first core shaft 11 is bent. The medical device with a sensor 1B of the third embodiment as described above can also achieve the same effects as those of the first embodiment. If the wiring 30B is configured as shown in Fig. 14, the flexibility of the wiring 30B can be improved as compared to that of the first embodiment.

### <Fourth Embodiment>

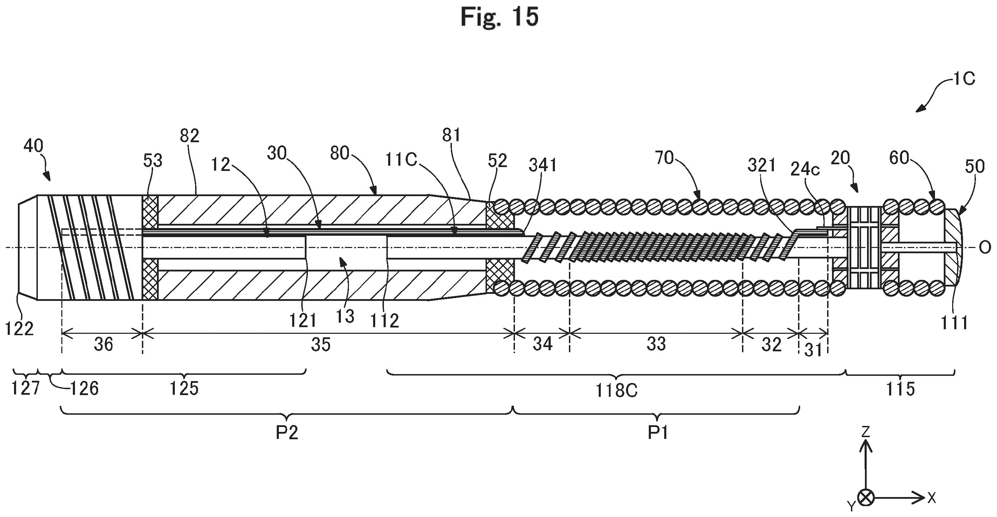

Fig. 15 is an explanatory view illustrating the configuration of a medical device with a sensor 1C in accordance with a fourth embodiment. The medical device with a sensor 1C of the fourth embodiment comprises a first core shaft 11C instead of the first core shaft 11 in the configuration described in the first embodiment. The first core shaft 11C has a fourth portion 118C instead of the second portion 116, the third portion 117, and the fourth portion 118 described in the first embodiment.

The fourth portion 118C is a portion provided on the more proximal end side than the first portion 115 and next to the first portion 115. The fourth portion 118C is a portion having the largest outer diameter in the first core shaft 11, and has a substantially cylindrical shape with a substantially constant outer diameter. The core shaft 11C does not have a tapered portion. In the present embodiment, the first roll portion 32 of the wiring 30 is located on a portion of the distal end side of the fourth portion 118C of the first core shaft 11C. The second roll portion 33 of the wiring 30 is located in a portion near the center of the fourth portion 118C of the first core shaft 11C. The third roll portion 34 of the wiring 30 is located on a portion of the proximal end side of the fourth portion 118C of the first core shaft 11C. In other words, the roll portions 32, 33, 34 of the wiring 30 are all located on the fourth portion 118C and are not located on the tapered portion.

As such, the configuration of the medical device with a sensor 1C can be modified in various ways. The core shaft 11C may not have a tapered portion. The wiring 30 may not be wound around the tapered portion. The first core shaft 11C may not have the first portion 115. Even in the medical device with a sensor 1C of the fourth embodiment described above, the wiring 30 can be moved in gap portions in the first roll portion 32 and the third roll portion 34 when the first core shaft 11C is bent. Thus, the fourth embodiment can achieve the same effects as the first embodiment.

### <Fifth Embodiment>

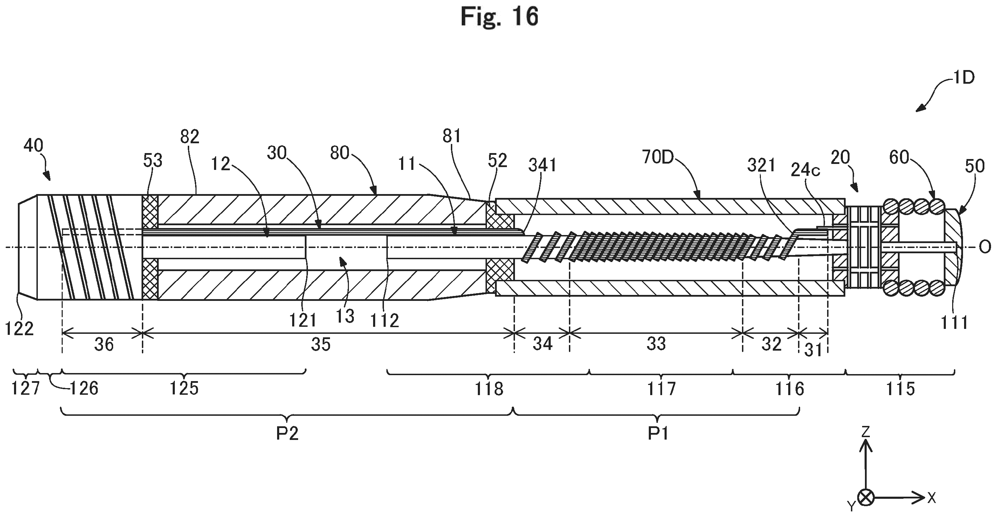

Fig. 16 is an explanatory view illustrating the configuration of a medical device with a sensor 1D in accordance with a fifth embodiment. The medical device with a sensor 1D of the fifth embodiment comprises a distal end side tube 70D instead of the second coil 70 in the configuration described in the first embodiment.

The distal end side tube 70D is positioned on the more proximal end side than the sensor 20. The distal end side tube 70D surrounds the outer peripheries of the second portion 116, the third portion 117 and a portion of the distal end side of the fourth portion 118 in the first core shaft 11. It is preferable that the distal end side tube 70D has antithrombotic ability, flexibility, and biocompatibility, and can be formed of resin or metal material. As the resin material, at least any one of polyimide resins, polyamide resins, polyolefin resins, polyester resins, polyurethane resins, silicone resins, fluororesins can be adopted, for example. As the metal material, at least any one of stainless steel, nickel-titanium alloys, and cobalt-chromium alloys may be adopted, for example. The distal end portion of the distal end side tube 70D is secured to the sensor 20 by the first joint part 51. The proximal end portion of the distal end side tube 70D is secured to the first core shaft 11 and tube 80 by the second joint part 52.

In this way, the configuration of the medical device with a sensor 1D can be modified in various ways. The medical device with a sensor 1D may not comprise the second coil 70 described in the first embodiment. The medical device with a sensor 1D may be configured not to comprise neither the second coil 70 nor the first coil 60. If the first coil 60 is omitted, the first portion 115 of the first core shaft 11 which is located on the more distal end side than the sensor 20 may be omitted. The medical device with a sensor 1D of the fifth embodiment as described above can also achieve the same effects as those of the first embodiment.

### <Sixth Embodiment>

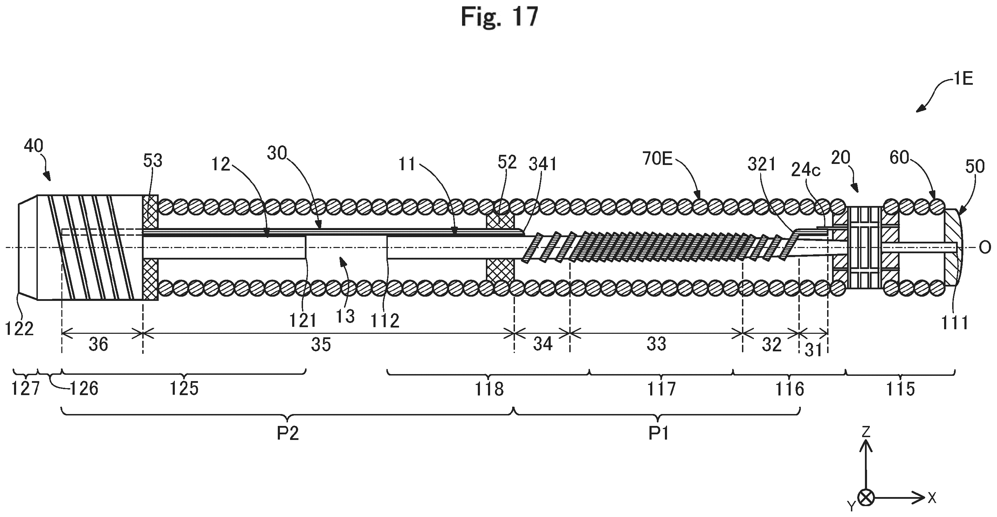

Fig. 17 is an explanatory view illustrating the configuration of a medical device with a sensor 1E in accordance with a sixth embodiment. The medical device with a sensor 1E of the sixth embodiment comprises a second coil 70E instead of the second coil 70 and does not comprise the tube 80 in the configuration described in the first embodiment.

The second coil 70E surrounds the outer peripheries of the second portion 116, third portion 117 and fourth portion 118 of the first core shaft 11. The second coil 70E further surrounds the outer periphery of a portion of the distal end side of the fifth portion 125 in the second core shaft 12. The second coil 70E extends from the proximal end of the sensor 20 to the distal end of the connector 40. The distal end portion of the second coil 70E is secured to the sensor 20 by the first joint part 51. The middle portion of the second coil 70E is secured to the first core shaft 11 by the second joint part 52. The proximal end portion of the second coil 70E is secured to the connector 40 and the second core shaft 12 by the third joint part 53. The second coil 70E covers a portion of the proximal end side of the first core shaft 11 and a portion of the distal end side of the second core shaft 12 to connect the first core shaft 11 to the second core shaft 12.

In this manner, the configuration of the medical device with a sensor 1E can be modified in various ways. The medical device with a sensor 1E may not have the tube 80. In the medical device with a sensor 1E, the first core shaft 11 and the second core shaft 12 may be connected by the second coil 70E. The second coil 70E may be divided into two or more coils positioned in a row in the X-axis direction. In this case, two or more coils may be formed from different materials. The medical device with a sensor 1E of the sixth embodiment as described above can also achieve the same effects as those of the first embodiment.

### <Seventh Embodiment>

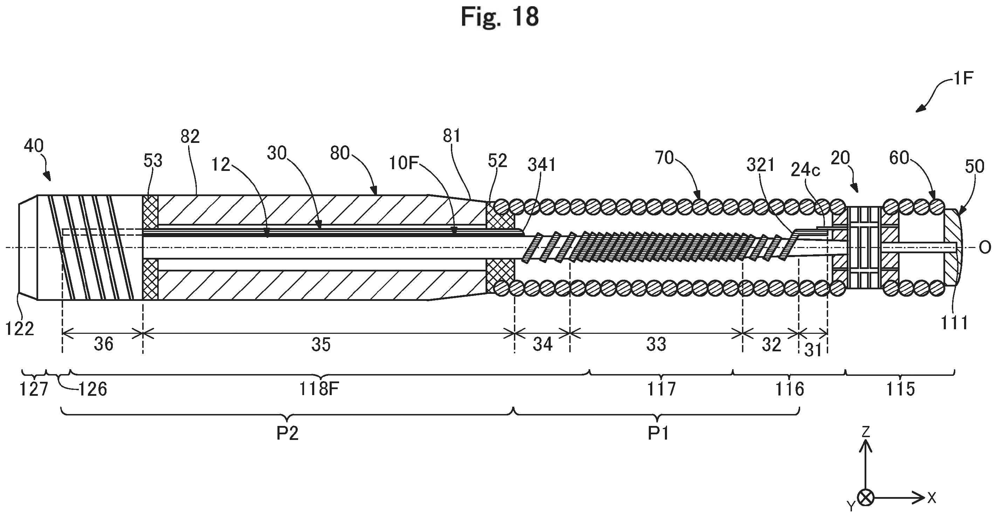

Fig. 18 is an explanatory view illustrating the configuration of a medical device with a sensor 1F in accordance with a seventh embodiment. The medical device with a sensor 1F of the seventh embodiment comprises a core shaft 10F instead of the first core shaft 11 and the second core shaft 12 in the configuration described in the first embodiment.

The core shaft 10F is a solid member which has an elongated shape extending along the X-axis direction. The core shaft 10F comprises the first portion 115, the second portion 116, the third portion 117, a fourth portion 118F, the sixth portion 126, and the seventh portion 127 from the distal end 111 toward the proximal end 112 in order. The configuration of each of the portions 115, 116, 117, 126, 127 excluding the fourth portion 118F is as described in the first embodiment. The fourth portion 118F of the core shaft 10F is a portion provided between the third portion 117 and the sixth portion 126. The fourth portion 118F is a substantially cylindrical portion having a substantially constant outer diameter.

In this manner, the configuration of the medical device with a sensor 1F can be modified in various ways. The medical device with a sensor 1F may be configured to have a single core shaft 10F only. In other words, the medical device with a sensor 1F may be configured to have no gap 13 provided between the first core shaft 11 and the second core shaft 12. The medical device with a sensor 1F of the seventh embodiment as described above can also achieve the same effects as those of the first embodiment. According to the medical device with a sensor 1F of the seventh embodiment, since the gap 13 is absent, the rigidity of the medical device with a sensor 1F can be improved further.

### <Eighth Embodiment>

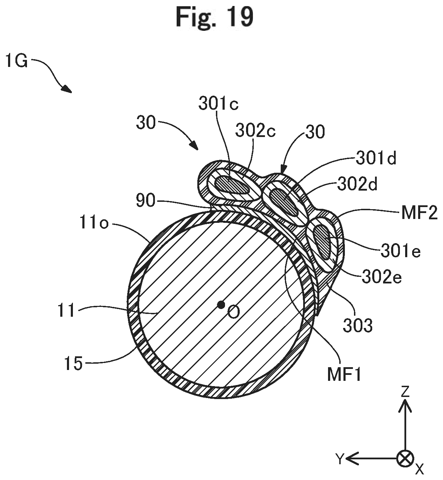

Fig. 19 is a view illustrating an example of the transverse section in the line A-A of Fig. 10 in accordance with an eighth embodiment. The medical device with a sensor 1G of the eighth embodiment further comprises an adhesive layer 90 in the configuration described in the first embodiment. In the medical device with a sensor 1G, the wiring 30 is secured to the first core shaft 11 by welding. The adhesive layer 90 is formed between the wiring 30 and the first core shaft 11. The adhesive layer 90 secures the wiring 30 to the first core shaft 11. The adhesive layer 90 is formed by heat treatment while the wiring 30 is wound around the first core shaft 11 to elute the adhesive layer for bonding the spacings of the wiring together onto the first core shaft 11 side and then solidify the adhesive layer. The range in the axial direction over which the adhesive layer 90 is formed is at least a portion of the range over which the wiring 30 is wound.

In this way, the configuration of the medical device with a sensor 1G can be modified in various ways. The medical device with a sensor 1G may have the adhesive layer 90. One adhesive layer 90 may be provided, or two or more adhesive layers 90 may be provided in a range where the wiring 30 is wound. The medical device with a sensor 1G of the eighth embodiment as described above can also achieve the same effects as those of the first embodiment. According to the medical device with a sensor 1G of the eighth embodiment, at least a portion of the wiring 30 can be secured to the first core shaft 11 by the adhesive layer 90. Since the adhesive layer 90 is formed by eluting the adhesive layer for bonding the spacings of the wiring together onto the first core shaft 11 side and then solidifying the adhesive layer, the wiring 30 and the first core shaft 11 can be secured without use of separate adhesives.

### <Modified Examples of Present Embodiments>

The present disclosure is not limited to the above embodiments, and can be implemented in various aspects without departing from the gist thereof. For example, the following modified examples are also possible.

### [Modified Example 1]

The configurations of the medical devices with a sensor 1, 1A-1G have been illustrated in the above first to eighth embodiments. However, the configuration of the medical device with a sensor can be modified in various ways. For example, the sensor 20 is not limited to a sensor that measures the electrical resistance of bodily fluids such as blood flowing within a blood vessel. The sensor 20 may be a sensor that measures blood pressure, i.e., intravascular pressure. The measured blood pressure can be used to derive coronary flow reserve. Coronary flow reserve is also referred to as fractional flow reserve, or FFR for short. Coronary flow reserve is pressure at the proximal end of stenosis versus pressure at the distal end of stenosis, and can be used as an indicator of severity of physiological stenosis. For example, the sensor 20 may not be formed using the sheet-form sensor sheet 24. The sensor 20 may be configured to secure any number of sensor elements to the outer peripheral surface of the tubular member 22 in any manner.

For example, in the medical device with a sensor, at least a portion of the first coil 60, the second coil 70, the second coil 70E, the distal end side tube 70D, and the tube 80 may be covered with resin coatings. In the medical device with a sensor, the entire body except the connector 40 may be covered with resin coatings. As coating resins, any one of hydrophilic resins and hydrophobic resins can be used. For example, an inner coil may be provided within the first coil 60.

For example, the first core shaft 11 and the second core shaft 12 may be positioned without the gap 13 being made therebetween. In other words, the proximal end face of the first core shaft 11 may come into contact with the proximal end face of the second core shaft 12. For example, the first core shaft 11 may not pass through the sensor 20 in the X-axis direction. In this case, the distal end 111 of the first core shaft 11 may be secured to the sensor 20 by the first joint part 51.

For example, the proximal end face of the first tube 41a and the distal end face of the sixth tube 41f may not be inclined with respect to the X-axis direction; they may be perpendicular to it. For example, the both end faces of tubes 41b-41e may not be inclined with respect to the X-axis direction; they may be perpendicular to it. For example, the seventh portion 127 of the second core shaft 12 may not protrude from the proximal end of the connector 40. For example, the second core shaft 12 may not pass through the insulators 43a-43e within the connector 40. In this case, the proximal end 122 of the second core shaft 12 is located at the distal end of the connector 40. The third joint part 53 connects the proximal end portion of the second core shaft 12 to the distal end of the connector 40.

### [Modified Example 2]

In the above first to eighth embodiments, the configurations of the wiring 30, 30A, 30B have been illustrated. However, various modifications can be made to the configurations of the wiring. For example, the number of leads 301a-301e included in the wiring may be one, or any number of two or more. For example, leads may not be tied together in the wiring. For example, the wound direction of the wiring may be the same as that of the second coil 70.

For example, Fig. 9 has illustrated the configuration in which the inner coating portions 302a-302e and the outer coating portion 303 have been entirely removed in the circumferential direction of the leads 301a-301e. In a portion of the leads 301a-301e, the inner coating portions 302a-302e and the outer coating portion 303 may be removed only partly in the circumferential direction.

For example, the wiring may not be secured to the first core shaft 11 at the distal end portion 321 of the first roll portion 32. The wiring may not be secured to the first core shaft 11 at the proximal end portion 341 of the third roll portion 34. The wiring may be secured to the first core shaft 11 at a location other than the distal end portion 321 and the proximal end portion 341. The number and positions of locations where the wiring is secured to the first core shaft 11 may be determined arbitrarily.

### [Modified Example 3]

The configurations of the medical device with a sensor 1, 1A-1D of the first to eighth embodiments, and each configuration of the above modified examples 1, 2 may be combined appropriately. For example, the medical device with a sensor 1A of the second embodiment may be configured to have the wiring 30B described in the third embodiment. For example, the medical device with a sensor may be configured to combine at least a part of the wiring 30A described in the second embodiment, the wiring 30B described in the third embodiment, the first core shaft 11C described in the fourth embodiment, the distal end side tube 70D described in the fifth embodiment, the second coil 70E described in the sixth embodiment, the core shaft 10F described in the seventh embodiment, and the adhesive layer 90 described in the eighth embodiment.

The present aspects have been described above based on the embodiments and modified examples. The embodiments of aspects described above are for ease of understanding of the present aspects, and do not limit the present aspects. The present aspects may be modified or improved without departing from the spirit thereof and claims, and the present aspects include their equivalents. Unless such technical features are explained as essentials in the present specification, they can be appropriately deleted.

## Claims

1. A medical device with a sensor (1, 1A-1G), comprising:
a sensor (20);
a core shaft (11, 11C); and
wiring (30, 30A, 30B) with a transverse sectional shape in which the thickness direction is smaller than the width direction, wherein a lead (301a-301e) electrically connected to the sensor (20) are positioned inside the wiring (30, 30A, 30B), the wiring (30, 30A, 30B) being wound around the core shaft (11, 11C) in a predetermined range of the long axis direction of the core shaft (11, 11C) in a state where the thickness direction continuously follows the radial direction of the core shaft (11, 11C).

2. The medical device with a sensor (1, 1A-1B, 1D-1G) according to claim 1,
wherein the core shaft (11) comprises, in at least a part of the predetermined range, tapered portions (116, 117) tapering in outer diameter from a proximal end toward a distal end.

3. The medical device with a sensor (1, 1A-1B, 1D-1G) according to claim 2,
wherein the wiring (30, 30A, 30B) is wound around the tapered portions (116, 117) of the core shaft (11).

4. The medical device with a sensor (1, 1A-1C, 1E-1G) according to any one of claims 1 to 3, further comprising:
coils (60, 70) covering at least a part of the core shaft (11, 11C) in the long axis direction of the core shaft (11, 11C).

5. The medical device with a sensor (1, 1A-1C, 1E-1G) according to claim 4,
wherein a wound direction of the coil (70) is opposite to a wound direction of the wiring (30, 30A, 30B).

6. The medical device with a sensor (1, 1A-1E, 1G) according to any one of claims 1 to 5,
wherein the core shaft is a first core shaft (11, 11C), and
wherein the medical device further comprises a second core shaft (12) positioned on the more proximal end side than the first core shaft (11, 11C) with a gap (13) being made and positioned between the distal end of the second core shaft (12) and the proximal end of the first core shaft (11, 11C).

7. The medical device with a sensor (1, 1A-1D, 1G) according to claim 6, further comprising:
a tube (80) covering a portion of the first core shaft (11, 11C) and at least a portion of the second core shaft (12), and connecting the first core shaft (11, 11C) to the second core shaft (12).

8. The medical device with a sensor (1, 1A-1G) according to any one of claims 1 to 7,
wherein the lead is a first lead (301a), and
wherein the wiring (30, 30A, 30B) has a second lead (301b) tied together with the first lead (301a).

9. The medical device with a sensor (1, 1A-1G) according to any one of claims 1 to 8,
wherein the wiring (30, 30A, 30B) has a pair of opposing main surfaces and a pair of opposing side surfaces, and
wherein the wiring (30, 30A, 30B) is wound around the core shaft (11, 11C) in a state where a first main surface of the pair of main surfaces continuously faces an outer peripheral surface of the core shaft (11, 11C).

10. The medical device with a sensor (1, 1A-1G) according to any one of claims 1 to 9,
wherein the wiring (30, 30A, 30B) is slidable with respect to the core shaft (11, 11C) in at least a part of the predetermined range.

11. The medical device with a sensor (1, 1A-1G) according to any one of claims 1 to 10,
wherein the wiring (30, 30A, 30B) further has coating portions (302a-302e, 303) covering outer peripheral surfaces of the lead (301a-301e).

12. The medical device with a sensor (1, 1A-1G) according to any one of claims 1 to 11,
wherein the core shaft (11, 11C) passes through the sensor (20) in the long axis direction.

13. The medical device with a sensor (1G) according to any one of claims 1 to 12, further comprising:
an adhesive layer (90) bonding the core shaft (11) to the wiring (30).
